# EUROPEAN PATENT SPECIFICATION

(11) **EP 3 613 842 B1**
(45) Date of publication and mention of the grant of the patent: **19.10.2022**
(21) Application number: 18768702.5
(22) Date of filing: 13.03.2018
(51) Int. Cl.: C12N 1/20, C12P 19/62, A61K 31/7048, A61P 33/14, C12R 1/465

(54) **IVERMECTIN B1B PRODUCING STRAIN AND USE THEREOF**
IVERMECTIN-B1B-PRODUZIERENDER STAMM UND VERWENDUNG DAVON
SOUCHE PRODUCTRICE D'IVERMECTINE B1B ET UTILISATION ASSOCIÉE

(30) Priority: 13.03.2017 CN 201710145887
(43) Date of publication of application: 26.02.2020
(73) Proprietor: Zhejiang Hisun Pharmaceutical Co., Ltd., Taizhou, Zhejiang 318000 (CN)
(72) Inventor: HUANG, Jun, Taizhou Zhejiang 318000 (CN); LI, Na, Taizhou Zhejiang 318000 (CN); LIN, Jiatan, Taizhou Zhejiang 318000 (CN); LI, Meihong, Taizhou Zhejiang 318000 (CN); ZHOU, Jun, Taizhou Zhejiang 318000 (CN); CHANG, Zongming, Taizhou Zhejiang 318000 (CN)
(74) Representative: HGF
(86) International application number: PCT/CN2018/078773
(87) International publication number: WO 2018/166427

(56) References cited:
- EP-A1- 3 118 305
- WO-A1-2015/135242
- CN-A- 1 687 403
- CN-A- 101 613 712
- CN-A- 103 834 605
- RU-C1- 2 156 064
- US-A- 4 199 569
- JANINA DEMELER ET AL: "Measuring the effect of avermectins and milbemycins on somatic muscle contraction of adult Haemonchus contortus and on motility of Ostertagia circumcincta in vitro", PARASITOLOGY, vol. 141, no. 7, 27 June 2014 (2014-06-27) , pages 948-956, XP055747218, GB ISSN: 0031-1820, DOI: 10.1017/S0031182013002291
- ZHANG XIAOLIN ET AL: "Construction of ivermectin producer by domain swaps of avermectin polyketide synthase in", APPLIED MICROBIOLOGY AND BIOTECHNOLOGY, SPRINGER BERLIN HEIDELBERG, BERLIN/HEIDELBERG, vol. 72, no. 5, 16 May 2006 (2006-05-16), pages 986-994, XP037057214, ISSN: 0175-7598, DOI: 10.1007/S00253-006-0361-2 [retrieved on 2006-05-16]
- SABINE GAISSER ET AL: "Direct production of ivermectin-like drugs after domain exchange in the avermectin polyketide synthase of Streptomyces avermitilis ATCC31272", ORGANIC & BIOMOLECULAR CHEMISTRY, vol. 1, no. 16, 1 January 2003 (2003-01-01), page 2840, XP55747478, ISSN: 1477-0520, DOI: 10.1039/b304022d
- LI M ET AL: "Engineering of avermectin biosynthetic genes to improve production of ivermectin in Streptomyces avermitilis", BIORGANIC & MEDICINAL CHEMISTRY LETTERS,, vol. 18, no. 20, 15 October 2008 (2008-10-15), pages 5359-5363, XP025562061, ISSN: 0960-894X, DOI: 10.1016/J.BMCL.2008.09.061 [retrieved on 2008-09-19]
- MENG LI ET AL: "Enhancement of avermectin and ivermectin production by overexpression of the maltose ATP-binding cassette transporter in", BIORESOURCE TECHNOLOGY, ELSEVIER, AMSTERDAM, NL, vol. 101, no. 23, 28 June 2010 (2010-06-28), pages 9228-9235, XP028320939, ISSN: 0960-8524, DOI: 10.1016/J.BIORTECH.2010.06.132 [retrieved on 2010-07-10]

## Description

### FIELD

The present invention belongs to the field of biomedicine, and relates to a strain of *Streptomyces avermitilis* with high yield of ivermectin B1b and a method for producing ivermectin B1b using the strain.

### BACKGROUND

Ivermectin has an effective killing effect on both internal and external parasites, especially nematodes and arthropods. It has stronger permeability and safety for mammalian body tissues and has a longer duration. It is especially suitable for the control of parasites in muscles, organs and special tissues that are difficult to be reached by general oral anthelmintic. It has a special effect on the gastrointestinal nematodes and parasites. It is recognized as a multi-component antibiotic with broad spectrum, high efficiency, safety, little residue and no drug resistance.

So far, there are two main methods for producing ivermectin according to domestic and foreign reports. One of the methods is to chemically reduce avermectin B1 by hydrogenation to obtain ivermectin. The chemical method needs to separate and purify avermectin B1 from the fermentation broth of *Streptomyces avermitilis* and then change it to ivermectin by reduction reaction. The production cost of the process is relatively high, and the yield of ivermectin B1b is only 15%. There are many impurities in the chemical synthesized ivermectin B1b obtained from avermectin B1, and the purification process is complicated, and it is difficult to isolate a large amount of ivermectin B1b. The other method is to use genetically engineered strain to produce ivermectin. For example, CN200510074936.X discloses a method of obtaining a engineered strain producing ivermectin by replacing the coding region of the avermectin polyketide synthase DH2-KR2 domain of *Streptomyces avermitilis* with the coding region of polyketide synthase DH-ER-KR domain, and the yield of ivermectin B1a is about 1-4 µg/mL, but the yield of ivermectin B1b is not mentioned. CN200910089970.2 discloses the expression plasmid of malEFG gene encoding maltose transporter. The plasmid was transformed into *Streptomyces avermitilis* and the maltose transporter overexpression recombinant strain was constructed. The recombinant strain is used for the overexpression of a maltose transport system gene to improve the utilization ratio of maltose. This method increases the yield of ivermectin from about 10 µg/mL to about 35 µg/mL. PCT Applications WO2015135242A1 and WO2015135467A1 disclose the constructing process of a genetically engineered strain MA220, which is obtained by a two-step genetic modification from *Streptomyces avermitilis* MA-4680: first, the aveD gene in MA4680 is inactivated by PCR targeting technology to obtain a genetically engineered strain AD28 which does not produce avermectin A component but only produces avermectin B component; then, the aveA1 gene is replaced with the milA1 gene of *Streptomyces milbemycinicus* by genetic recombination technology to obtain recombinant *Streptomyces* MA220. The fermentation product of recombinant *Streptomyce* MA220 contains a very small amount of ivermectin B1b component in addition to the main components of tenvermectin A (TEVA) and tenvermectin B (TEVB). The above-mentioned method for producing ivermectin by genetic engineering to construct a new strain either has relatively high requirements on experimental conditions and skill level of researchers, or the fermentation yield of the genetic engineering strain is unsatisfactory, so this method basically remains at the laboratory stage, which is not conducive to the formation of industrialization.

In summary, the yield of ivermectin in the conventional art is low, and the product is either a mixture of ivermectin B 1a and B1b, or ivermectin B1a, and no attention has been paid to the production of ivermectin B1b as the single component. Therefore, there is an urgent need to find new microbial strains for producing ivermectin, especially ivermectin B1b, and a preparation method thereof, which can give a satisfactory yield.

The inventors unexpectedly screened and obtained a high-yield strain which can be directly used for fermenting ivermectin B1b, and further provide a method for producing ivermectin B1b using the strain. On one hand, ivermectin B1b can be effectively produced, and on the other hand, it can lay a foundation for further research on the biological activity of ivermectin B1b and structural modification.

### SUMMARY

One of the objects of the present disclosure is to provide a novel high-yield strain capable of producing ivermectin B1b to solve the above-mentioned problems.

One aspect of the present disclosure is to provide a high-yield microorganism strain for ivermectin B1b. The strain is *Streptomyces avermitilis* strain C63-51, which was deposited on December 22, 2016 in the China General Microbiological Culture Collection Center (address: No.1 Courtyard, Beichen West Road, Chaoyang District, Beijing, China Institute of Microbiology, Chinese Academy of Sciences) with an accession number of CGMCC NO. 13370 and registered and confirmed to be viable.

Another aspect of the present disclosure is to provide a use of *Streptomyces avermitilis* strain C63-51 in the manufacture of ivermectin B1b or a pharmaceutical composition containing ivermectin B1b.

Another aspect of the present disclosure is to provide a method of producing ivermectin B1b using *Streptomyces avermitilis* strain C63-51. The method comprises performing fermentation in a culture medium containing an assimilable carbon source and/or nitrogen source by using *Streptomyces avermitilis* strain C63-51.

The present disclosure is also directed to a composition comprising *Streptomyces avermitilis* strain C63-51 of the present disclosure. The composition may be a composition further comprising a strain other than the strain C63-51; preferably, the other strain may be a strain for producing ivermectin B1b, such as *Streptomyces avermitilis, Streptomyces hygroscopicus,* genetically engineered strain MA220, etc., especially genetically engineered strain MA220.

The present disclosure is also directed to a use of the above-mentioned composition in the manufacture of ivermectin B1b or a pharmaceutical composition comprising ivermectin B1b.

Also described herein is an antiparasitic composition comprising ivermectin B1b produced by the method of the present disclosure.

Preferably, the above-mentioned assimilable carbon source is one or more of sucrose, glucose, amylase, fructose, rhamnose, raffinose, xylose, arabinose, industrial molasses, lactose, galactose, maltose, trehalose, xylan, dextrin, corn starch, sorbitol, salicin, inositol, mannitol, glycerol, glycine or inulin.

Preferably, the assimilable nitrogen source is one or more of beef extractum, yeast extractum, yeast extract, yeast powder, peptone, tryptone, gluten powder, cottonseed meal, peanut meal, soybean meal, dried powder of corn steep liquor, bran, urea, ammonium salt or nitrate.

Preferably, the culture medium further comprises an inorganic salt, and the inorganic salt is one or more of MnSO₄, Na₂MoO₄ ,CoCl₂, CaCO₃, ZnSO₄, FeSO₄, (NH₄)₂SO₄, FeCl₃, KNO₃, KH₂PO₄, K₂HPO₄, MgCl₂, MgSO₄, NaCl, CuSO₄, NiSO₄ or KCl.

Preferably, the culture medium contains corn starch 120-160 g/L, amylase 0.2 g/L, soybean meal 10-40 g/L, peanut meal 0-10 g/L, yeast powder 5-20 g/L, (NH₄)₂SO₄ 0-4 g/L, MnSO₄ 0.024 g/L, Na₂MoO₄ 0.024 g/L, CoCl₂ 0.01-0.04 g/L, CaCO₃ 3-7 g/L.

Preferably, the culture medium contains corn starch 140 g/L, amylase 0.2 g/L, soybean meal 20 g/L, peanut meal 5 g/L, yeast powder 10 g/L, (NH₄)₂SO₄ 2 g/L, MnSO₄ 0.024 g/L, Na₂MoO₄ 0.024 g/L, CoCl₂ 0.02 g/L, CaCO₃ 7 g/L.

Preferably, the temperature of the fermentation is 20 to 40 °C, more preferably 25 to 30 °C, most preferably 28 °C. Preferably, the pH condition of the fermentation is controlled in the fermentation method of the present disclosure, and the pH is 6.0-8.0, more preferably 6.5-7.5, most preferably 7.0-7.2. The duration of the fermentation of the method of the present disclosure may be 288-312 hours; the ventilation of the method of the present disclosure may be 0.6-1.1 vvm.

In the present disclosure, the "ventilation" is the amount of filter-sterilized air passing through each unit volume of culture medium every minute.

In the present disclosure, the *"Streptomyces avermitilis* strain C63-51", "strain C63-51", and "mutagenized strain C63-51" have the same meaning.

In the present disclosure, the "genetically engineered strain MA220", "strain MA220", "tenvermectin producing strain MA220", "recombinant Streptomyces MA220", and "original strain MA220" have the same meaning.

In the present disclosure, ivermectin B1b may be detected by the following HPLC method:
chromatographic column: C18 column, 5 µm, 4.6 × 250 mm;
mobile phase: methanol - acetonitrile - water = 81 : 7 : 12 (volume ratio);
flow rate: 1 ml/min;
detection wavelength: 240 nm;
sample injection volume: 10 µl.

The present disclosure has at least the following advantages over the conventional art:
1. *Streptomyces avermitilis* strain C63-51 obtained by screening in the present disclosure can be directly used for the fermentation and production of ivermectin B1b. Compared with the chemical preparation of ivermectin, it avoids complicated process steps of separation and purification and chemical reduction, so that greatly reduces production cost.
2. In the conventional art, for example, in the method of CN200510074936.X, the yield of ivermectin B1a is about 1-4 µg/mL, but the yield of ivermectin B1b is not mentioned; in the method of CN200910089970.2, the yield of ivermectin is about 35 µg/mL, the product is a mixture of ivermectin B1a and B1b, and it is relatively difficult to separate ivermectin B1b; in the methods of WO2015135242A1 and WO2015135467A1, the titer of ivermectin B1b produced by the original strain MA220 is 200mg/L. In the present disclosure, the ivermectin B1b produced by *Streptomyces avermitilis* strain C63-51 in shake flask fermentation can reach 6500 mg/L, and the titer in 50 L fermenter can reach 4000 mg/L. The yield of the ivermectin B1b of the present disclosure is much higher than that of the conventional art; moreover, the content of ivermectin B1b in the fermentation broth of *Streptomyces avermitilis* strain C63-51 exceeds 60% with little impurities, high titer, and the purification process is simple.

### BRIEF DESCRIPTION OF DRAWINGS

Figure 1 shows the comparison of fermentation products of *Streptomyces avermitilis* strain C63-51 and original strain MA220: A is the HPLC chromatogram of the fermentation broth produced by the original strain MA220 in Example 1; B is the HPLC chromatogram of the fermentation broth produced by *Streptomyces avermitilis* strain C63-51 in Example 1.
Figure 2 shows the mass spectrum of ivermectin B1b prepared in Example 5.

### DETAILED DESCRIPTION

In the following examples, unless otherwise specified, the used reagents and instruments are all commonly used in the art, and can be purchased from chemical or biological products/preparation companies; the methods used in the following examples are all conventional methods in the art, and those having ordinary skill in the art can know the operation of these experiments and obtain corresponding results without any doubt according to the conventional art or the operation manual provided by the manufacturer.

### Example 1 Screening of Streptomyces avermitilis strain C63-51

### 1. Nitrosoguanidine (NTG) mutagenesis of strain MA220

The tenvermectin producing strain MA220 (the preparation method thereof is described in WO2015135242A1 and WO2015135467A1) was the original strain. A fresh culture slant of the original strain MA220 was taken, and a spore suspension was prepared with 0.1 mol/L phosphate buffer solution of pH 6.0. The spore suspension was treated with NTG with a final concentration of 1 mg/ml, and shaken at 34 °C for 1 hour. The spores were washed three times with saline, diluted and then applied to YMS solid medium (yeast extract 4 g/L, glucose 4 g/L, malt extract 10 g/L, trace element solution 5 ml/L, agar 20 g/L), and cultured at 28 °C for 4 days. A single colony with relatively large diameter and high spore production was picked and transferred to YMS solid culture medium plate, and cultured at 28 °C for 7 days. According to the fermentation verification method described below, about 600 mutagenized strains were screened and mutant strain 58-46 was obtained, which has an ivermectin B1b titer of 1600 mg/L.

### 2. Atmospheric and room temperature plasma (ARTP) mutagenesis of mutant strain 58-46

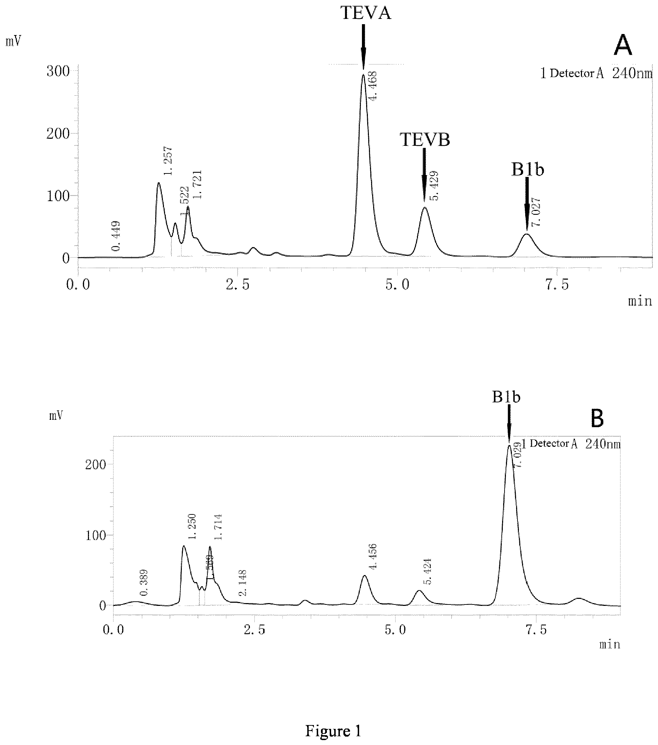

The above-mentioned mutant strain 58-46 mutagenized by NTG was used as the starting strain, and the mature spores on the fresh culture were picked, washed with saline, and the spore suspension was prepared. 10 µl of spore suspension was added to a sterilized microscope slide and placed in a plasma chamber for irradiation. The irradiation conditions were set as follows: helium gas as the generator, flow rate: 12.5 L/min, the distance between the plasma torch nozzle exit and the sample plate: 2 mm, the irradiation power: 100 W, and the irradiation time: 30 seconds. After the irradiation, the sample slide was taken out with sterile tweezers, placed in a 2 ml sterile EP tube containing saline, shaken vigorously for 2 minutes using a vortex mixer, and the sample on the slide was thoroughly washed and suspended in saline. After dilution by gradient, the sample was applied to YMS solid culture medium plate and cultured at 28 °C for 4 days. A single colony with relatively large diameter and high spore production was picked and transferred to YMS solid culture medium plate, and cultured at 28 °C for 7 days. According to the fermentation verification method described below, about 600 mutagenized strains were screened and a high-yield strain capable of producing ivermectin B1b was obtained and named *Streptomyces avermitilis* strain C63-51. The titer of ivermectin B1b produced by the original strain MA220 was 200 mg/L with lots of impurities; the titer of ivermectin B1b produced by *Streptomyces avermitilis* strain C63-51 was increased to 3000 mg/L with less impurities. The result is shown in Figure 1, wherein A is the HPLC chromatogram of the fermentation broth of the original strain MA220, and B is the HPLC chromatogram of the fermentation broth of *Streptomyces avermitilis* strain C63-51.

### 3. Fermentation verification method

### (1) Seed medium preparation and cultivation

Seed medium formula (g/L): corn starch 25, soybean meal 8, peanut meal 10, yeast powder 9.5, CoCl₂ 0.03, pH 7.2, sterilized at 121 °C for 20 minutes. About 1 cm² of the lawn were scraped with an inoculating shovel to a seed culture medium from the cultured spores, and the culture temperature was 28 °C, 250 rpm, cultured for 40 hours in a shaker. At this time, the culture solution had a pH of 6.86 and a mycelium concentration of 35% by volume.

### (2) Fermentation medium preparation and cultivation

Fermentation medium formula (g/L): corn starch 120, amylase 0.2, soybean meal 10, yeast powder 5, MnSO₄ 0.024, Na₂MoO₄ 0.024, CoCl₂ 0.01, CaCO₃ 3, pH 7.2, sterilized at 121 °C for 20 minutes. The inoculum size was 6% by volume. The culture temperature was 28 ° C, 250 rpm, cultured for 12 days in a shaker. At this time, the culture solution had a pH of 6.60 and a mycelium concentration of 33% by volume.

### (3) Determination of the titer of the fermentation broth by high performance liquid chromatography (HPLC)

Extraction of fermentation broth: 1 ml of fermentation broth was collected, 4 ml of anhydrous methanol was added, subjected to ultrasonication for 1 h and then filtered. The filtrate was used directly for HPLC analysis. The HPLC analysis conditions were: chromatographic column, C18 Hypersil ODS2 4.6×250×5 (Dalian Elliott); mobile phase, methanol: acetonitrile: water = 81:7:12 (volume ratio); flow rate, 1 ml/min; detection wavelength, 240nm.

### Example 2 Morphology and cultivation characteristics of Streptomyces avermitilis strain C63-51

Experiments were carried out with reference to the contents of the *Streptomyces Identification Manual,* the *Classification and Identification of Actinomycetes,* and the *Manual for Identification of Common Bacterial.* The seven culture media ISP2, ISP3, ISP4, Gauze's No.1, Glucose Aspartate, LB and YMS were used for the test of the morphological and cultivation characteristics of the strains. After incubation at 28 °C for 5 to 7 days, the color and pigment of the mycelium were observed. The result is shown in Table 1.

**Table 1 Culture characteristics of strain C63-51 on 7 different media**

| Medium | Growth Situation | Substrate Mycelium | Aerial Mycelium | Soluble Pigment |
|---|---|---|---|---|
| ISP2 | 4 | Light Brown | Cinnamon | Light Yellow Brown |
| ISP3 | 3 | Light Yellow | Yellow White | None |
| ISP4 | 3 | Yellow | Light Yellow | Light Yellow Brown |
| Gauze's No.1 | 4 | Beige | Milky | Yellow |
| Glucose Aspartate | 4 | Yellow Orange | Yellow Orange | Light Yellow Tan |
| LB | 2 | Beige | Beige | Yellow |
| YMS | 4 | Yellow | Yellow | Yellow |

### Example 3 Physiological and biochemical characteristics of Streptomyces avermitilis strain C63-51

(1) Utilization of carbon source: ISP9 was used as the basic medium, and the final concentration of each carbon source was 1.0% (mass percent concentration). The result is shown in Table 2.
(2) Utilization of nitrogen source: the culture medium with a formula of KH₂PO₄ 1.36g/L, Na₂HPO₄ 2.13g/L, MgSO₄ 0.2 g/L, CaCl₂ 0.005 g/L, glucose 10 g/L, FeSO₄ 0.0005 g/L was used as the basic medium. The concentrations of potassium nitrate and ammonium sulfate were both 0.1% (mass percent concentration). The result is shown in Table 2.

**Table 2 Utilization of carbon and nitrogen sources of strain C63-51**

| Carbon Source | Growth Situation | Carbon Source | Growth Situation | Carbon Source | Growth Situation | Inorganic Nitrogen Source | Growth Situation |
|---|---|---|---|---|---|---|---|
| D-Glucose | 4 | Maltose | 4 | Inositol | 3 | Ammoniu m sulfate | + |
| D-Raffinose | 3 | D-Fructose | 4 | Mannitol | 4 | Potassium nitrate | - |
| D-Xylose | 3 | D-Sucrose | 3 | Glycine | 0 | | |
| D-Sorbitol | 4 | Salicin | 3 | Xylan | 3 | | |
| L-Arabinose | 4 | D-Lactose | 4 | Inulin | 2 | | |
| Glycerol | 4 | Galactose | 3 | Rhamnose | 4 | | |

(3) Degradation test and NaCl tolerance test: the basic medium used in the degradation test was GYEA (pH 6.8), and the results of various degradation tests are shown in Table 3. The NaCl tolerance test result showed that the strain has good tolerance to NaCl, and it could grow in the presence of 7% NaCl.

**Table 3 Degradation test results of strain C63-51**

| Degradation Product | Concentration of Degradation Product | Result | Degradation Product | Concentration of Degradation Product | Result |
|---|---|---|---|---|---|
| Adenine | 0.5% | 4, + | Tyrosine | 1.0% | 4, + |
| Guanine | 0.5% | 4, - | Tween -40 | 1.0% | 3, - |
| Hypoxanthine | 0.4% | 4, - | Tween-80 | 1.0% | 4, - |
| Casein | 1.0% | 4, + | | | |

(4) Physiological and biochemical tests, pH test and temperature test: the result of physiological and biochemical test is shown in Table 4. Both the pH test and the temperature test were carried out using Gauze's No.1 medium. The results showed that the strain could grow between 14 °C and 37 °C, and the optimum growth temperature was 28 °C; and it could grow between pH of 6.0 to 8.0, and the optimum range was 6.5 to 7.5.

**Table 4 Main physiological and biochemical characteristics of strain C63-51**

| Test Item | Result | Test Item | Result | Test Item | Result |
|---|---|---|---|---|---|
| Gelatin Liquefaction | - | Milk Peptonization | + | Cellulose Utilization | - |
| Starch Hydrolysis | + | Nitrate Reduction | + | | |
| Milk Solidification | + | Hydrogen Sulfide Production | + | | |

| | | | | | |
|---|---|---|---|---|---|
| Note: The numbers and symbols in Tables 1-4 represent: 0: no growth; 1: weak growth; 2: growth; 3: good growth; 4: best growth; +: positive; -: negative. | | | | | |

### Example 4 16S rDNA sequence analysis and strain identification

Fresh cells of the test strain C63-51 were collected, and the total DNA template was extracted by the lysozyme-modified Pitcher method (*Letters in Applied Microbiology,* 1989, 8: 151-156), and 16S rDNA gene amplification was carried out using universal primers (27F and 1495R).The PCR product was tested and purified, and subjected to sequencing directly (sequencing was carried out by Genscript Biotechnology (Nanjing) Co., Ltd.). The determined 16S rDNA sequence was compared with the sequences of related species and genus in the GenBank database to determine the taxonomic status of the strain.

The 16S rDNA sequence of strain C63-51 (CGMCC NO. 13370) was compared with related sequences in GenBank by BLAST. The results are shown in Table 5 (only the strains with relatively high homology are listed in the table).

**Table 5 Homology of strain C63-51 with related strains**

| Species | GenBank No. | Number of base differences | Homology (%) |
|---|---|---|---|
| *Streptomyces avermitilis* MA-4680 | NR - 074747.1 | 1491/1491 | 100% |
| *Streptomyces griseochromogenes* strain ATCC 14511 | CP016279.1 | 1476/1491 | 99% |
| *Streptomyces fimbriatus* | AB045868.1 | 1459/1476 | 99% |
| *Streptomyces plumbeus* strain S14 | HQ850409.1 | 1468/1490 | 99% |

Strain C63-51 (CGMCC NO. 13370) was found to have 100% homology with *Streptomyces avermitilis* via 16S rDNA region sequencing. In addition, phenomenal characteristics test of strain C63-51 (CGMCC NO. 13370) was carried out. The strain was found to be very similar to the classification parameters of *Streptomyces avermitilis,* so the strain C63-51 (CGMCC NO. 13370) was identified as *Streptomyces avermitilis.*

### Example 5 Optimization of Fermentation Medium

### (1) Fermentation medium optimization and cultivation

The fermentation medium in Example 1 was used as the basic medium. The concentration of each component in the formula was adjusted, the fermentation medium was optimized, and the formula was optimized to be (g/L): corn starch 120-160, amylase 0.2, soybean meal 10-40, peanut meal 0-10, yeast powder 5-20, (NH₄)₂SO₄ 0-4, MnSO₄ 0.024, Na₂MoO₄ 0.024, CoCl₂ 0.01-0.04, CaCO₃ 3-7, pH 7.2, sterilized at 121 °C for 20 minutes. The inoculum size was 6% by volume. The culture temperature was 28 °C, 250 rpm, and the culture was carried out for 12 days in a shaker.

### (2) Determination of the titer of the fermentation broth by high performance liquid chromatography (HPLC)

Extraction of fermentation broth: 1 ml of fermentation broth was taken, 4 ml of anhydrous methanol was added, and the mixture was filtered after a one-hour ultrasonication. The filtrate was used directly for HPLC analysis. The HPLC analysis conditions were: chromatographic column, C18 Hypersil ODS2 4.6×250×5 (Dalian Elite); mobile phase, methanol : acetonitrile : water = 81 : 7 : 12 (volume ratio); flow rate, 1 ml/min; absorption wavelength, 240nm.

The titer of the fermentation broth of different fermentation media was determined as described above, and the preferred fermentation medium formulation was determined according to the titer of the fermentation broth. The most preferred fermentation medium formula is (g/L): corn starch 140, amylase 0.2, soybean meal 20, peanut meal 5, yeast powder 10, (NH₄)₂SO₄ 2, MnSO₄ 0.024, Na₂MoO₄ 0.024, CoCl₂ 0.02, CaCO₃ 7, pH 7.2, sterilized at 121 °C for 20 minutes. The culture temperature was 28 °C, 250 rpm, and the culture was carried out for 12 days in a shaker. At this time point, the pH of the culture broth was 6.65 and the mycelium concentration was 37% by volume. The titer of ivermectin B1b produced by *Streptomyces avermitilis* strain C63-51 was increased to 6500 mg/L, which was 2 times higher than the original formulation.

### Example 6 Fermentation, isolation and preparation of ivermectin B1b

### (1) The slant strain cultivation

YMS medium was used for preparing the agar slant (g/L): yeast extract 4, glucose 4, malt extract 10, trace element solution 5 ml/L, agar 20, distilled water 1000 ml, pH 7.2. The mixture was sterilized at 121 °C for 20 minutes, cooled to 50-60 °C and used to prepare the culture slant. The strain C63-51 was inoculated on the slant, cultured at 28 °C for 7 days.

### (2) Seed medium preparation and cultivation

Seed medium formula (g/L): corn starch 25, soybean meal 8, peanut meal 10, yeast powder 9.5, CoCl₂ 0.03, pH 7.2, sterilized at 121 °C for 20 minutes. About 1 cm² of the culture with spores were scraped with an inoculating shovel to a seed culture medium, and the culture was carried out at temperature 28 °C, 250 rpm for 40 hours in a shaker. At this time, the pH of the culture broth was 6.86 and the mycelium concentration was 35%.

### (3) Preparation of seed medium in a tank

10 L seed medium was placed in a 15 L tank, sterilized by steam at 121 ° C for 30 minutes. 300 ml seed culture from a shake flask was inoculated into the tank. The culture temperature was 28 ± 1 °C, the stirring speed was 100-250 rpm, the ventilation was 1.0 vvm and the culture was carried out for 18 hours. At this time, the pH of the culture broth was 6.80 and the mycelium concentration was 30% (volume percentage).

### (4) Fermenter culture medium preparation and cultivation

The formula of the fermentation medium was: corn starch 140, amylase 0.2, soybean meal 20, peanut meal 5, yeast powder 10, (NH₄)₂SO₄ 2, MnSO₄ 0.024, Na₂MoO₄ 0.024, CoCl₂ 0.02, CaCO₃ 7, fermenter volume 50 L, feed volume 30 L, pH 7.2, sterilized by steam at 121 °C for 30 minutes and then cooled. 3 L of seed culture from a shake flask was inoculated into the fermenter. The temperature for culture was 28 ± 1 °C, the stirring speed was 101-308 rpm, the ventilation was 0.6-1.1 vvm and the culture was carried out for 12 days. The fermenter was discharged, and the fermentation unit of ivermectin B1b was measured to be 4000 mg/L.

### (5) Fermentation broth extraction

The obtained fermentation broth was filtered with a filter cloth to obtain a filter cake, and the filter cake was extracted twice with ethanol and combined to obtain an ethanol extract. The ethanol extract was concentrated until dry via vacuum concentration and extracted with ethyl acetate to obtain an extract containing ivermectin B1b. After mixed with silica gel, the extract was applied to a silica gel column and eluted with a gradient of petroleum ether/acetone at volume ratios of 90:10, 80:20, 70:30, 60:40, and the eluted fractions were collected respectively, detected by TLC, to obtain a component containing ivermectin B1b. The component was concentrated until dry via vacuum concentration to obtain a sample containing ivermectin B1b. The sample was subjected to reversed-phase chromatography separation under the following conditions:
Liquid phase system: Agilent 1100 semi-preparative high pressure liquid chromatography
Column: ZORBAXEclipse XDB-C18 (250 mm × 9.4 mm)
Eluent: methanol: acetonitrile : water = 46 : 46 : 8 (volume ratio)
Flow rate: 1.5 ml/min
Detection wavelength: λ=240nm

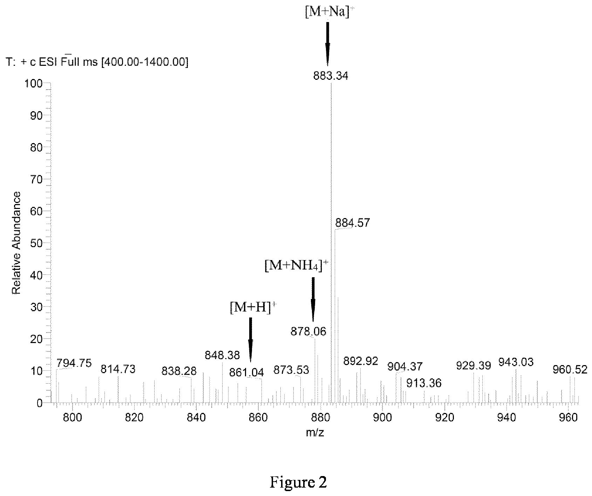

A compound with a peak at a time of 26.79 minute was collected. The compound was subjected to mass spectrographic analysis, and the spectrum is shown in Figure 2. The result showed that the molecular weight of the compound was 860, and it was determined to be ivermectin B1b, and the structure was as follow:

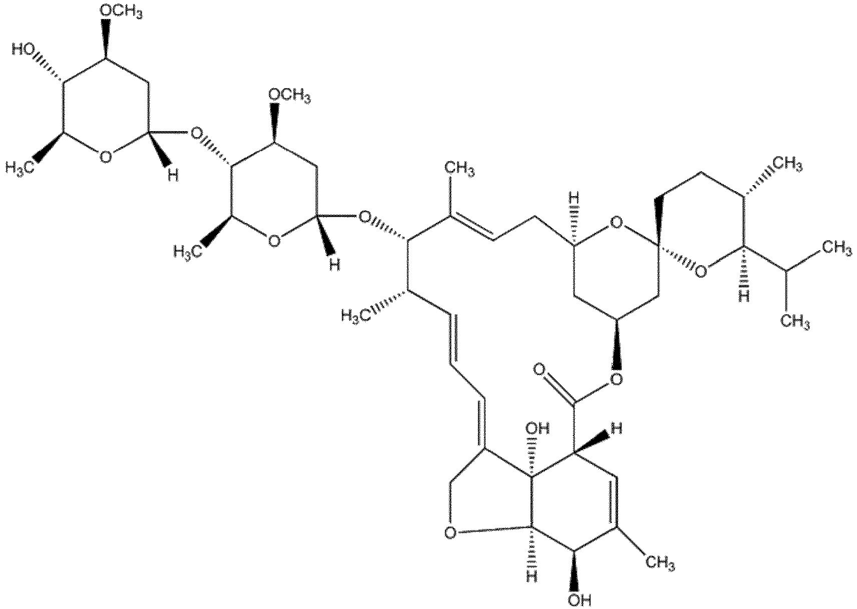

In summary, *Streptomyces avermitilis* strain C63-51 of the present disclosure is a high-yield strain for ivermectin B1b, and the shake flask fermentation unit can reach 6500 mg/L, the fermentation unit in 50 L fermenter can reach 4000 mg/L.

## Claims

1. A *Streptomyces avermitilis* strain C63-51, deposited on December 22, 2016 in the China General Microbiological Culture Collection Center (CGMCC) with an accession number CGMCC NO. 13370.

2. A composition comprising the *Streptomyces avermitilis* strain C63-51 according to claim 1.

3. Use of the *Streptomyces avermitilis* strain C63-51 according to claim 1 or the composition according to claim 2 in the manufacture of ivermectin B1b or a pharmaceutical composition containing ivermectin B1b.

4. A method of producing ivermectin B1b comprising performing fermentation in a medium containing an assimilable carbon source and/or nitrogen source using the *Streptomyces avermitilis* strain C63-51 according to claim 1.

5. The method according to claim 4, wherein the assimilable carbon source is one or more of sucrose, glucose, amylase, fructose, rhamnose, raffinose, xylose, arabinose, industrial molasses, lactose, galactose, maltose, trehalose, xylan, dextrin, corn starch, sorbitol, salicin, inositol, mannitol, glycerol, glycine or inulin.

6. The method according to claim 4 or 5, wherein the assimilable nitrogen source is one or more of beef extractum, yeast extractum, yeast extract, yeast powder, peptone, tryptone, gluten powder, cottonseed meal, peanut meal, soybean meal, dried powder of corn steep liquor, bran, urea, ammonium salt or nitrate.

7. The method according to any one of claims 4 to 6, wherein the medium further comprises an inorganic salt, and the inorganic salt is one or more of MnSO₄, Na₂MoO₄ ,CoCl₂, CaCO₃, ZnSO₄, FeSO₄, (NH₄)₂SO₄, FeCl₃, KNO₃, KH₂PO₄, K₂HPO₄, MgCl₂, MgSO₄, NaCl, CuSO₄, NiSO₄ or KCl.

8. The method according to any one of claims 4 to 7, wherein the medium contains corn starch 120-160 g/L, amylase 0.2 g/L, soybean meal 10-40 g/L, peanut meal 0-10 g/L, yeast powder 5-20 g/L, (NH₄)₂SO₄ 0-4 g/L, MnSO₄ 0.024 g/L, Na₂MoO₄ 0.024 g/L, CoCl₂ 0.01-0.04 g/L and CaCO₃ 3-7 g/L.

9. The method according to any one of claims 4 to 8, wherein the medium contains corn starch 140 g/L, amylase 0.2 g/L, soybean meal 20 g/L, peanut meal 5 g/L, yeast powder 10 g/L, (NH₄)₂SO₄ 2 g/L, MnSO₄ 0.024 g/L, Na₂MoO₄ 0.024 g/L, CoCl₂ 0.02 g/L and CaCO₃ 7 g/L.

10. The method according to any one of claims 4 to 9, wherein the temperature of the fermentation is 20 to 40 °C.

11. The method according to any one of claims 4 to 10, wherein the pH of the medium is 6.0-8.0.

12. The method according to any one of claims 4 to 11, wherein the duration of the fermentation is 288-312 hours, and/or with a ventilation of 0.6-1.1 vvm.

13. The method according to any one of claims 4 to 10, wherein the temperature of the fermentation is 25 to 30 °C, preferably 28 °C; the pH of the medium is 6.5-7.5, preferably 7.0-7.2.

## Patentansprüche

1. Stamm C63-51 von *Streptomyces avermitilis,* hinterlegt am 22. Dezember 2016 im China General Microbiological Culture Collection Center (CGMCC) mit einer Hinterlegungsnummer CGMCC NO. 13370.

2. Zusammensetzung, umfassend den Stamm C63-51 von *Streptomyces avermitilis* nach Anspruch 1.

3. Verwendung des Stamms C63-51 von *Streptomyces avermitilis* nach Anspruch 1 oder der Zusammensetzung nach Anspruch 2 bei der Herstellung von Ivermectin B1b oder einer pharmazeutischen Zusammensetzung, die Ivermectin B1b enthält.

4. Verfahren zur Herstellung von Ivermectin B1b, umfassend ein Ausführen einer Fermentation in einem Medium, das eine assimilierbare Kohlenstoffquelle und/oder Stickstoffquelle enthält, unter Verwendung des Stamms C63-51 von *Streptomyces avermitilis* gemäß Anspruch 1.

5. Verfahren nach Anspruch 4, wobei die assimilierbare Kohlenstoffquelle eine oder mehrere von Saccharose, Glucose, Amylase, Fructose, Rhamnose, Raffinose, Xylose, Arabinose, Industriemelasse, Lactose, Galactose, Maltose, Trehalose, Xylan, Dextrin, Maisstärke, Sorbit, Salicin, Inosit, Mannit, Glycerin, Glycin oder Inulin ist.

6. Verfahren nach Anspruch 4 oder 5, wobei die assimilierbare Kohlenstoffquelle eine oder mehrere von Rindfleischextrakt, extrahierte Hefe, Hefeextrakt, Hefepulver, Pepton, Trypton, Glutenpulver, Baumwollsamenmehl, Erdnussmehl, Sojabohnenmehl, getrocknetem Pulver aus Maisquellflüssigkeit, Kleie, Harnstoff, Ammoniumsalz oder Nitrat ist.

7. Verfahren nach einem der Ansprüche 4 bis 6, wobei das Medium ferner ein anorganisches Salz umfasst und das anorganische Salz eines oder mehrere von MnSO₄, Na₂MoO₄, CoCl₂, CaCO₃, ZnSO₄, FeSO₄, (NH₄)₂SO₄, FeCl₃, KNO₃, KH₂PO₄, K₂HPO₄, MgCl₂, MgSO₄, NaCl, CuSO₄, NiSO₄ oder KCl ist.

8. Verfahren nach einem der Ansprüche 4 bis 7, wobei das Medium Maisstärke 120-160 g/l, Amylase 0,2 g/l, Sojamehl 10-40 g/l, Erdnussmehl 0-10 g/l, Hefepulver 5-20 g/l, (NH₄)₂SO₄ 0-4 g/l, MnSO₄ 0,024 g/l, Na₂MoO₄ 0,024 g/l, CoCl₂ 0,01-0,04 g/l and CaCO₃ 3-7 g/l enthält.

9. Verfahren nach einem der Ansprüche 4 bis 8, wobei das Medium Maisstärke 140 g/l, Amylase 0,2 g/l, Sojamehl 20 g/l, Erdnussmehl 5 g/l, Hefepulver 10 g/l, (NH₄)₂SO₄ 2 g/l, MnSO₄ 0,024 g/l, Na₂MoO₄ 0,024 g/l, CoCl₂ 0,02 g/l and CaCO₃ 7 g/l enthält.

10. Verfahren nach einem der Ansprüche 4 bis 9, wobei die Temperatur der Fermentation 20 bis 40 °C ist.

11. Verfahren nach einem der Ansprüche 4 bis 10, wobei der pH des Mediums 6,0-8,0 ist.

12. Verfahren nach einem der Ansprüche 4 bis 11, wobei die Dauer der Fermentation 288-312 Stunden ist, und/oder mit einer Belüftung von 0,6-1,1 vvm.

13. Verfahren nach einem der Ansprüche 4 bis 10, wobei die Temperatur der Fermentation 25 bis 30 °C, vorzugsweise 28 °C ist; der pH des Mediums 6,5-7,5, vorzugsweise 7,0-7,2 ist.

## Revendications

1. Souche de *Streptomyces avermitilis* C63-51, déposée le 22 décembre 2016 au Centre général de collecte de cultures microbiologiques de Chine (CGMCC) avec un numéro de dépôt CGMCC No. 13370.

2. Composition comprenant la souche de *Streptomyces avermitilis* C63-51 selon la revendication 1.

3. Utilisation de la souche de *Streptomyces avermitilis* C63-51 selon la revendication 1 ou composition selon la revendication 2 dans la fabrication d'ivermectine B1b ou d'une composition pharmaceutique contenant de l'ivermectine B1b.

4. Procédé de production d'ivermectine B1b comprenant la réalisation d'une fermentation dans un milieu contenant une source de carbone et/ou une source d'azote assimilable utilisant la souche de *Streptomyces avermitilis* C63-51 selon la revendication 1.

5. Procédé selon la revendication 4, dans lequel la source de carbone assimilable est un ou plusieurs parmi le sucrose, le glucose, l'amylase, le fructose, le rhamnose, le raffinose, le xylose, l'arabinose, les molasses industrielles, le lactose, le galactose, le maltose, le tréhalose, le xylane, la dextrine, l'amidon de maïs, le sorbitol, la salicine, l'inositol, le mannitol, le glycérol, la glycine ou l'inuline.

6. Procédé selon la revendication 4 ou 5, dans lequel la source d'azote assimilable est un ou plusieurs parmi l'extractum de bœuf, l'extractum de levure, l'extrait de levure, la poudre de levure, la peptone, la tryptone, la poudre de gluten, le tourteau de coton, le tourteau d'arachide, le tourteau de soja, la poudre séchée de liqueur de macération du maïs, le son, l'urée, un sel d'ammonium ou le nitrate.

7. Procédé selon l'une quelconque des revendications 4 à 6, dans lequel le milieu comprend en outre un sel inorganique, et le sel inorganique est un ou plusieurs parmi MnSO₄, Na₂MoO₄ ,CoCl₂, CaCO₃, ZnSO₄, FeSO₄, (NH₄)₂SO₄, FeCl₃, KNO₃, KH₂PO₄, K₂HPO₄, MgCl₂, MgSO₄, NaCl, CuSO₄, NiSO₄ ou KCl.

8. Procédé selon l'une quelconque des revendications 4 à 7, dans lequel le milieu contient de l'amidon de maïs 120-160 g/L, de l'amylase 0,2 g/L, du tourteau de soja 10-40 g/L, du tourteau d'arachide 0-10 g/L, de la poudre de levure 5-20 g/L, (NH₄)₂SO₄ 0-4 g/L, MnSO₄ 0,024 g/L, Na₂MoO₄ 0,024 g/L, CoCl₂ 0,01-0,04 g/L et CaCO₃ 3-7 g/L.

9. Procédé selon l'une quelconque des revendications 4 à 8, dans lequel le milieu contient de l'amidon de maïs 140 g/L, de l'amylase 0,2 g/L, du tourteau de soja 20 g/L, du tourteau d'arachide 5 g/L, de la poudre de levure 10 g/L, (NH₄)₂SO₄ 2 g/L, MnSO₄ 0,024 g/L, Na₂MoO₄ 0,024 g/L, CoCl₂ 0,02 g/L et CaCO₃ 7 g/L.

10. Procédé selon l'une quelconque des revendications 4 à 9, dans lequel la température de fermentation est comprise entre 20 et 40 °C.

11. Procédé selon l'une quelconque des revendications 4 à 10, dans lequel le pH du milieu est compris entre 6,0 et 8,0.

12. Procédé selon l'une quelconque des revendications 4 à 11, dans lequel la durée de la fermentation est comprise entre 288 et 312 heures, et/ou avec une ventilation comprise entre 0,6 et 1,1 vvm.

13. Procédé selon l'une quelconque des revendications 4 à 10, dans lequel la température de fermentation est comprise entre 25 et 30 °C, de préférence de 28 °C ; le pH du milieu est compris entre 6,5 et 7,5, de préférence compris entre 7,0 et 7,2.
